# EUROPEAN PATENT SPECIFICATION

(11) **EP 4 588 477 B1**
(45) Date of publication and mention of the grant of the patent: **27.05.2026**
(21) Application number: 23941824.7
(22) Date of filing: 01.12.2023
(51) Int. Cl.: A61K 31/485, A61K 31/519, A61P 25/22, A61P 25/24, A61P 25/30, A61P 25/36, A61K 9/00

(54) **PHARMACEUTICAL COMPOSITION COMPRISING NALTREXONE AND RISPERIDONE FOR USE IN PREVENTING CANNABIS RELAPSE**
PHARMAZEUTISCHE ZUSAMMENSETZUNG ENTHALTEND NALTREXON UND RISPERIDON ZUR ANWENDUNG BEI DER VORBEUGUNG EINES CANNABIS-RÜCKFALLS
COMPOSITION PHARMACEUTIQUE COMPRENANT DE LA NALTREXONE ET DE LA RISPÉRIDONE POUR UNE UTILISATION DANS LA PRÉVENTION DE LA RECHUTE AU CANNABIS

(43) Date of publication of application: 23.07.2025
(73) Proprietor: Shenzhen Sciencare Pharmaceutical Co., Ltd., Shenzhen, Guangdong 518118 (CN)
(72) Inventor: QU, Wei, Shenzhen Guangdong 518118 (CN); HUANG, Yamin, Shenzhen Guangdong 518118 (CN); YIN, Shugui, Shenzhen Guangdong 518118 (CN); ZHANG, Tao, Shenzhen Guangdong 518118 (CN); ZHAO, Xiaoge, Shenzhen Guangdong 518118 (CN); WANG, Yunhua, Shenzhen Guangdong 518118 (CN); GUO, Anni, Shenzhen Guangdong 518118 (CN); QIU, Xinmin, Shenzhen Guangdong 518118 (CN)
(74) Representative: karo IP
(86) International application number: PCT/CN2023/135922
(87) International publication number: WO 2025/112048

(56) References cited:
- WO-A1-2009/029308
- WO-A2-2008/095086
- CN-A- 112 245 434
- CN-A- 115 518 048
- HANEY MARGARET ET AL: "Naltrexone Maintenance Decreases Cannabis Self-Administration and Subjective Effects in Daily Cannabis Smokers", vol. 40, no. 11, 16 April 2015 (2015-04-16), Cham, pages 2489 - 2498, XP093263961, ISSN: 0893-133X, Retrieved from the Internet <URL:https://pmc.ncbi.nlm.nih.gov/articles/PMC4569951/pdf/npp2015108a.pdf> DOI: 10.1038/npp.2015.108
- NATSUKO NARITA ET AL: "YM90K, a selective-amino-3-hydroxy5-methyl-4-isoxazole propionate (AMPA) receptor antagonist, prevents induction of heat shock protein HSP -70 and hsp -70 mRNA in rat retrosplenial cortex by phencyclidine", ADDICTION BIOLOGY, ABINGDON CARFAX PUBLISHING, ABINGDON, GB, vol. 2, no. 1, 9 June 2006 (2006-06-09), pages 47 - 56, XP072052088, ISSN: 1355-6215, DOI: 10.1080/13556219772859
- GORELICK DAVID A: "Pharmacological Treatment of Cannabis-Related Disorders: A Narrative Review", CURRENT PHARMACEUTICAL DESIGN, BENTHAM SCIENCE PUBLISHERS, NL, vol. 22, 1 January 2016 (2016-01-01), NL , pages 6409 - 6419, XP093319140, ISSN: 1381-6128, DOI: 10.2174/1381612822666160822
- WANG, CHUNYUN): "Effects of Risperidone on Affective Disorder, Obsessive Compulsive Disorder and Schizophrenia with Depressive Symptom", CHINESE JOURNAL OF TISSUE ENGINEERING RESEARCH JUNE, vol. 8, no. 21, 25 July 2004 (2004-07-25), pages 4346 - 4347, XP009564061, ISSN: 2095-4344

## Description

### Field of the Invention

The present application relates to the technical field of medicines, and particularly to a pharmaceutical composition for preventing relapse into cannabis use and application thereof. An active pharmaceutical ingredient in the pharmaceutical composition includes naltrexone and risperidone.

### Background

Cannabis originally derived from extracts of the cannabis plants, is a chemically complex mixture that was once used clinically as an anesthetic. However, due to its potential for addiction and non-negligible side effects, it was later abandoned. Currently, it is primarily traded as a drug. Short-term use of cannabis may cause adverse reactions such as sedation, hyperemia, tachycardia, cough due to lung irritation, hyperphagia, and hypotension. Long-term and high-dose use of cannabis may lead to cognitive and memory disorders, resulting in loss of memory, anxiety, hallucination, depression, and even psychosis, which is one of the reasons why cannabis depressants have been abandoned in clinical practice. Studies have confirmed that the use of cannabis can trigger hidden mental diseases and exacerbate personality disorders in patients with diagnosed schizophrenia. Additionally, studies have shown that cannabis has a significant inhibition effect on the immune system and lung function, potentially induces respiratory diseases such as large airway inflammation, chronic bronchitis, and pneumonia, and may also increase the risk of developing invasive testicular cancer.

Modern pharmacological studies indicate that the main component in cannabis that induces addiction behavior is cannabinoids (exocannabinoid), which stimulate specific areas of the brain, thereby stimulating the corresponding reward systems and causing changes in brain structure and function. Although there have been many reports on drugs for the treatment and prevention of cannabis addiction, most of these drugs are used for detoxification treatment of addicted patients or as adjunctive treatment during the withdrawal stage, with results showing that the efficacy is not significant, and they are less used for preventing relapse and treating adverse symptoms caused by cannabis addiction. Clinically, relapse is generally considered a different treatment stage from the aforementioned addiction and withdrawal. Narcotic-addicted individuals are highly likely to start using the addictive substance they abused before withdrawal in the short term, and the adverse symptoms caused by addiction lead to great psychological trauma for the patients, which is an important factor in inducing relapse. The phenomenon of relapse is not only a common problem among narcotic users but also a challenge that narcotic control work has always hoped to solve

HANEY MARGARET ET AL ("Naltrexone Maintenance Decreases Cannabis Self-Administration and Subjective Effects in Daily Cannabis Smokers", NEUROPSYCHOPHARMACOLOGY, vol. 40, no. 11, 16 April 2015 (2015-04-16), pages 2489-2498), indicates that naltrexone may reduce relapse into cannabinoid use.

NATSUKO NARITA ET AL ("YM90K, a selective-amino-3-hydroxy5-methyl-4-isoxazole propionate (AMPA) receptor antagonist, prevents induction of heat shock protein HSP -70 and hsp -70 mRNA in rat retrosplenial cortex by phencyclidine",ADDICTION BIOLOGY, ABINGDON CARFAX PUBLISHING, ABINGDON, GB, vol. 2, no. 1, 9 June 2006 (2006-06-09), pages 47-56) teaches the combination of THC and lofexidine against cannabis relapse.

Therefore, there is an urgent need to develop a drug that effectively prevents relapse into cannabis use.

### Summary

The present invention is defined in the appended claims. Any references in the description to methods of treatment refer to the compounds, pharmaceutical compositions and medicaments of the present invention for use in a method for treatment of the human (or animal) body by therapy.

Based on this, according to various embodiments of the present application, a pharmaceutical composition for preventing relapse into cannabis use and application thereof are provided. The technical solutions are as follows.

One embodiment of the present application provides a pharmaceutical composition for preventing relapse into cannabis use, where an active pharmaceutical ingredient in the pharmaceutical composition includes naltrexone and risperidone.

A further embodiment of the present application provides application of the pharmaceutical composition in the preparation of a drug for preventing relapse into cannabis use.

A further embodiment of the present application provides application of the pharmaceutical composition in the preparation of a drug for treating adverse symptoms caused by cannabis addiction.

A further embodiment of the present application provides a method for preventing relapse into cannabis use, including administering a therapeutically effective amount of the pharmaceutical composition to a patient in need.

A further embodiment of the present application provides a method for treating adverse symptoms caused by cannabis addiction, including administering a therapeutically effective amount of the pharmaceutical composition to a patient in need.

Details of one or more embodiments of the present application are set forth in the following description, and other features, objects, and advantages of the present application will become apparent from the specification and claims thereof.

### Brief Description of the Drawings

In order to more clearly illustrate the technical solutions in the embodiments of the present application or in the related art, the drawings required in the descriptions of the embodiments or the related art will be briefly introduced below.

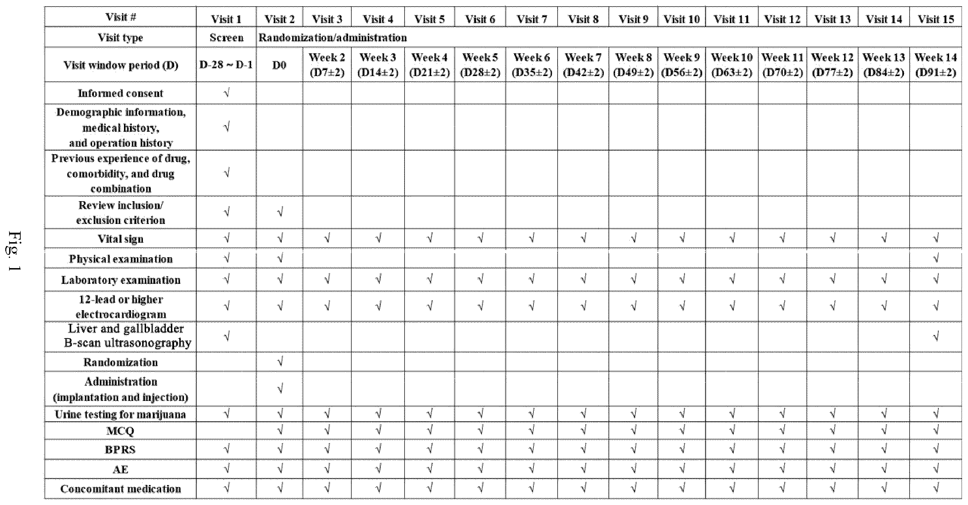

Fig. 1 is a flowchart of a subject trial studying a therapeutic effect of a pharmaceutical composition on adverse symptoms caused by cannabis addiction according to one embodiment of the present application.

### Detailed Description of the Embodiments

The pharmaceutical composition of the present application in the preparation of a drug for preventing relapse into cannabis use or a drug for treating adverse symptoms caused by cannabis addiction is further illustrated below in conjunction with embodiments and examples.

Unless defined otherwise, all technical and scientific terms used herein have the same meaning as commonly understood by a person skilled in the art to which the present application belongs. The terms used herein in the description of the present application are for the purpose of describing specific examples only and are not intended to limit the present application.

### Term

Unless otherwise stated or contradicted, the terms or phrases used herein have the following meanings.

As used herein, the terms "preferably", "preferred", "better", and the like are merely intended to describe embodiments or examples, and it is to be understood that they do not limit the scope of the present application.

In the present application, "further", "particular", and the like are used for descriptive purposes to indicate differences in content, but should not be construed to limit the scope of the present application.

In the present application, among the technical features described in an open manner, a closed technical solution consisting of the listed features is included, and an open technical solution containing the listed features is also included.

In the present application, where a numerical interval (i.e., numerical range) is recited, optional numerical distributions are considered to be continuous throughout the numerical interval, unless specifically stated otherwise, and two numerical endpoints (i.e., the minimum and maximum values) of the numerical range as well as numerical values between the numerical endpoints are included. Unless specifically stated otherwise, when a numerical interval indicates only integers within the numerical interval, two end integers of the numerical range as well as integers between the two end integers are included. In addition, when multiple ranges are provided to describe a feature or characteristic, these ranges may be combined. In other words, unless otherwise indicated, the ranges disclosed herein are to be understood to include any and all subranges subsumed therein.

In the present application, the weight may be in µg, mg, g, kg, and other mass units well known in the chemical field.

As used herein, "subject" and "patient" refer to an animal, preferably a mammal, and more preferably a human. The subject includes, but is not limited to, a patient with a disease, disorder and/or symptom. The term "mammal" mainly refers to warm-blood vertebrate mammals, including, but not limited to, cats, dogs, rabbits, bears, foxes, wolves, monkeys, deer, mice (such as rats and small mice), pigs, cattle, sheep, horses, and humans, preferably primates, and more preferably humans.

As used herein, "drug" includes any agent, compound, composition, or mixture that provides a physiological and/or pharmacological effect in vivo or in vitro, and often provides a beneficial effect. There is no particular limitation on the extent to which the "drug" may produce a physiological and/or pharmacological effect in vivo, either systemic or localized. There is no particular limitation on the activity of the "drug", which may be an active substance that interacts with other substances or an inert substance that does not interact.

As used herein, "pharmaceutically acceptable" refers to those ligands, materials, compositions and/or dosage forms which are, within the scope of reasonable medical determination, suitable for administration to a patient and are commensurate with a reasonable benefit/risk ratio.

As used herein, "pharmaceutically acceptable carrier" includes buffering agents, sterile water for injection, solvents, dispersion media, coatings, antibacterial agents, antifungal agents, isotonic agents, and absorption delaying agents that are compatible with pharmaceutical administration. Each must be "pharmaceutically acceptable" in the sense of being compatible with the other ingredients of the formulation and not injurious to the patient. Suitable examples include, but are not limited to: (1) sugar, such as lactose, glucose, and sucrose; (2) starch, such as corn starch, potato starch, and substituted or unsubstituted β-cyclodextrin; (3) cellulose and its derivative, such as carboxymethylcellulose sodium, ethyl cellulose, and cellulose acetate; (4) powdered tragacanth; (5) malt; (6) gelatin; (7) talc; (8) excipient, such as cocoa butter and suppository wax; (9) oil, such as peanut oil, cottonseed oil, safflower oil, sesame oil, olive oil, corn oil, and soybean oil; (10) glycol, such as propylene glycol; (11) polyol, such as glycerol, sorbitol, mannitol, and polyethylene glycol; (12) ester, such as ethyl oleate and ethyl laurate; (13) agar; (14) buffering agent, such as magnesium hydroxide and aluminum hydroxide; (15) alginic acid; (16) pyrogen-free water; (17) isotonic saline; (18) Ringer's solution; (19) ethanol; (20) phosphate buffering solution; and (21) other non-toxic compatible substances employed in pharmaceutical formulations.

The present application relates to application of a pharmaceutical composition in the preparation of a drug for treating relapse into cannabis use. An active pharmaceutical ingredient in the pharmaceutical composition includes naltrexone and risperidone.

Naltrexone is an opioid receptor non-selective antagonist that completely blocks the pharmacological effects of intravenous opioids (e.g., heroin) through competitive binding at the opioid receptor site. Risperidone is a selective monoaminergic antagonist that has a high affinity for serotonergic 5-HT2 receptors and dopamine D2 receptors and may improve positive symptoms of schizophrenia.

The superior efficacy of naltrexone in the clinical treatment of patients with opiate addiction has been demonstrated, but the efficacy of treatment for drug dependence or disease associated with cannabis addiction, particularly for the prevention of relapse into cannabis use, is unclear.

Through the experimental study in the present application, it is found that the compound composition of naltrexone and risperidone has an unexpectedly superior effect in preventing relapse into cannabis use. The inventors speculate that this is most likely related to the mechanism of cannabis addiction relapse.

The behavioral process of drug addiction is divided into three stages: (I) formation; (II) withdrawal (cessation of drug in a living environment) or regression (cessation of drug in a training environment; specific cue regression, environmental cue regression, and specific + environmental cue regression); (III) relapse (triggered by cues or low-dose drug exposure).
(I) Formation: in the formation of cannabis addiction, cannabinoids enter the body to increase dopamine levels by activating cannabinoid type 1 (CB1) receptors within the ventral tegmental area, thereby inducing their enhancing properties. Subsequently, compensatory adaptation has also occurred in some non-CB1 receptor systems, including dopamine receptor systems, 5-HT receptor systems, opioid receptor systems, and GABA receptor systems.
(II) Withdrawal: when chronic cannabis intake is stopped, somatic withdrawal syndrome occurs. Studies have shown that chronic treatment and subsequent withdrawal of cannabinoids leads to reduced neuronal activity in the mesolimbic dopaminergic pathway, which is involved in the long-term aversive consequences of drug withdrawal. It has also been shown that chronic tetrahydrocannabinol exposure alters several intracellular signaling processes that mediate physiological and behavioral changes during withdrawal, such as CB1, AC, cAMP, and PKA. After chronic tetrahydrocannabinol exposure, synaptic depression induced by decreased expression of CB1 receptors and changes in the activity of AC, cAMP, and PKA are observed, which may be related to the withdrawal response.
(III) Relapse: under baseline conditions, dopamine neurons within the ventral tegmental area are inhibited by activating GABAergic neurons. Upon presentation of drug-conditioned cues, dopamine neurons switch to a phasic firing pattern, activating CB1 receptors on the GABAergic neurons, inhibiting the GABAergic neurons, and thereby disinhibiting dopaminergic neurons to increase dopamine levels. Cues conditioned on cannabis smoking may induce a drug-seeking response (i.e., craving) by initiating a phasic dopamine event. Another dopamine-independent mechanism involved in drug-seeking responses involves endogenous cannabinoid/glutamate interactions in the corticostriatal connecting portion of the reward system.

The pharmaceutical composition of the present application may act to prevent relapse into cannabis use by participating in the regulation of neural pathways associated with the relapse behavior.

In some of these embodiments, the pharmaceutical composition includes a therapeutically effective amount of an active pharmaceutical ingredient, and the active pharmaceutical ingredient includes naltrexone and risperidone with a mass ratio of (5-30): 1.

In some of these embodiments, the pharmaceutical composition includes the following parts.

In some embodiments, the pharmaceutical composition includes: (a) naltrexone; (b) risperidone; and (c) a pharmaceutically acceptable carrier.

In some embodiments, the carrier is poly (D, L-) lactide.

In some embodiments, the pharmaceutical composition includes: (a) naltrexone with a concentration of 0.375-0.5 mg/mg; (b) risperidone with a concentration of 0.0375-0.05 mg/mg; and (c) poly (D, L-) lactide.

In some embodiments, the pharmaceutical composition of the present application is used together or separately with pharmaceutically acceptable auxiliary materials to prepare a clinically acceptable formulation.

In some embodiments, the formulation includes a liquid formulation and a solid formulation.

In some embodiments, the formulation is selected from any of the following groups or a suitable combination thereof:
1) tablets, capsules, granules, powders, dispersible powders, suspensions, or solutions; and
2) oral liquids, buccal agents, injections, suppositories, sprays, drops, or patches.

In some embodiments, the formulation is in the form of an implant.

The present application also relates to application of the pharmaceutical composition including a therapeutically effective amount of the above-mentioned active pharmaceutical ingredient in the preparation of a drug for preventing relapse into cannabis use.

The definitions of "pharmaceutical composition", "drug", "active pharmaceutical ingredient", and the like are consistent with the above.

In some embodiments, the therapeutically effective amount is 0.4-0.6 mg/mg. Further, it may be selected from 0.4 mg/mg, 0.45 mg/mg, 0.5 mg/mg, 0.55 mg/mg, 0.6 mg/mg, etc.

In some embodiments, the pharmaceutical composition includes 160 mg-170 mg of the active pharmaceutical ingredient. Further, it may be selected from 160 mg, 162 mg, 165 mg, 168 mg, 170 mg, etc.

According to the above-mentioned application, the present application also provides a method for preventing relapse into cannabis use, including administering a therapeutically effective amount of the pharmaceutical composition to a patient in need. That is, the patient is administered a therapeutically effective amount of the pharmaceutical composition.

### Mode of administration

The dosage form and mode of administration of the active pharmaceutical ingredient of the present application or the pharmaceutical composition thereof are not particularly limited. Representative modes of administration include, but are not limited to: oral, rectal, parenteral (intravenous, intramuscular, or subcutaneous) injection, and local administration.

Solid dosage forms for oral administration include capsules, tablets, pills, dispersible powders, and granules. In these solid dosage forms, an active compound is mixed with at least one conventional inert excipient (or carrier), such as sodium citrate or dicalcium phosphate, or with the following ingredients: (a) a filler or compatibilizer, such as starch, lactose, sucrose, glucose, mannitol, and silicic acid; (b) a binder, such as hydroxymethyl cellulose, alginate, gelatin, polyvinylpyrrolidone, sucrose, and acacia; (c) a humectant, such as glycerol; (d) a disintegrant, such as agar, calcium carbonate, potato or tapioca starch, alginic acid, certain complex silicates, and sodium carbonate; (e) a solubilizer, such as paraffin; (f) an absorption enhancer, such as, quaternary amine compound; (g) a wetting agent, such as cetyl alcohol and glyceryl monostearate; (h) an adsorbent, such as kaolin; and (i) a lubricant, such as, talc, calcium stearate, magnesium stearate, solid polyethylene glycol, sodium lauryl sulfate, or mixtures thereof. In capsules, tablets, and pills, the dosage forms may also include buffering agents. Solid dosage forms, such as tablets, sugar spheres, capsules, pills, and granules may be prepared with coatings and shells, such as sausage casings and other materials well known in the art. They may contain opacifying agents, and the active compound or compound in this composition may be released in a delayed manner in a part of the digestive tract. Examples of embedded compositions which may be used are polymeric substances and wax-like substances. The active compound may also form a microcapsule form, if desired, with one or more of the above-mentioned excipients.

Liquid dosage forms for oral administration include pharmaceutically acceptable emulsions, solutions, suspensions, syrups, or tinctures. In addition to the active compound, the liquid dosage forms may include inert diluents commonly used in the art, such as water or other solvents, solubilizer, and emulsifying agents, specifically such as, ethyl alcohol, isopropyl alcohol, ethyl carbonate, ethyl acetate, propylene glycol, 1,3-butanediol, dimethylformamide, and oils, particularly, cottonseed oil, peanut oil, corn germ oil, olive oil, castor oil, sesame oil, or mixtures of these substances. Besides these inert diluents, the composition may also include excipient, such as wetting agents, emulsifying agents, suspensions, sweeteners, flavoring agents, and fragrances. Suspensions, for example, may include suspending agents, in particular, ethoxylated isostearyl alcohol, polyoxyethylene sorbitol, sorbitan ester, microcrystalline cellulose, aluminum methoxide, and agar, or mixtures of these substances.

Compositions for parenteral injection may include physiologically acceptable sterile aqueous or non-aqueous solutions, dispersions, suspensions or emulsions, and sterile powders for redissolution into sterile injectable solutions or dispersions. Suitable aqueous or non-aqueous carriers, diluents, solvents, or excipients include water, ethanol, polyol, and suitable mixtures thereof.

Dosage forms for local administration include ointments, dispersible powders, patches, sprays, and inhalants. The active ingredient is mixed under sterile conditions with a pharmaceutically acceptable carrier and any preservatives, buffering agents, or propellants which may be required.

Different pharmaceutically acceptable auxiliary materials may be added to the drug of the examples of the present disclosure to prepare suitable clinical dosage forms including, but not limited to, those described above.

These pharmaceutically acceptable auxiliary materials include, but are not limited to, diluents, wetting agents, adhesives, disintegrants, lubricants, color-flavor-taste regulators, solvents, solubilizer, hydrotropy, emulsifying agents, antioxidants, metal complexing agents, inert gases, preservatives, local analgesics, pH regulators, isotonic regulators, etc. Further, diluents include, such as starch, sucrose, celluloses, and mineral salts; wetting agents include, such as water and ethanol; adhesives include, such as starch slurry, dextrin, sugars, cellulose derivatives, gelatin, povidone, and polyethylene glycol; disintegrants include, such as starch, carboxymethyl starch sodium, low-substituted hydroxypropyl cellulose, croscarmellose sodium, crospovidone, surfactants, and effervescent disintegrants; lubricants include, such as talc, calcium stearate, magnesium stearate, magnesium lauryl sulfate, aerosil and polyethylene glycol; color-flavor-taste regulators include, such as pigments, fragrances, sweeteners, mucilages, and deodorants, in particular magenta and xylitol; solvents include, such as water, oils, ethanol, glycerol, propylene glycol, polyethylene glycol, dimethyl sulfoxide, liquid paraffin, fatty oils and ethyl acetate; solubilizer include, such as Tweens, myrij, brij, soaps, sulfates, and sulfonates; hydrotropy include, such as organic acids (e.g., citric acid) and salts thereof, amide and amine compounds, inorganic salts, polyethylene glycol, povidone, and glycerol; emulsifiers include, such as spans, Tweens, myrij, brij, glycerol fatty acid esters, higher fatty acid salts, sulfates, sulfonates, acacia, tragacanth, gelatin, pectin, lecithin, agar, sodium alginate, hydroxides, silicon dioxide, and bentonite; suspending agents include, such as glycerol, syrup, acacia, tragacanth, agar, sodium alginate, cellulose derivatives, povidone, carbopol, polyvinyl alcohol, and thixotrope; antioxidants include, such as sulfites, pyrosulfite, bisulfite, ascorbic acid, and gallic acid and esters thereof; metal complexing agents include, such as ethylenediaminetetraacetic acid disodium salts and polycarboxylic acid compounds; inert gases include, such as nitrogen and carbon dioxide; preservatives include, such as parabens, organic acids and salts (e.g., sodium benzoate) thereof, quaternary ammonium compounds, chlorhexidine acetate, alcohols, phenols, and volatile oils; local analgesics include, such as benzyl alcohol, chlorobutanol, lidocaine, and procaine; pH regulators include, such as hydrochloric acid, sulfuric acid, phosphoric acid, tartaric acid, acetic acid, sodium hydroxide, sodium bicarbonate, ethylenediamine, meglumine, phosphate, acetate, citric acid, and citrate; isotonic or isotonic regulators include, such as glucose, sodium chloride, sodium citrate, sorbitol, and xylitol. It will be appreciated that the diluents described in the examples herein may also be referred to as filling agents and play the same role in pharmaceutical formulations. The water described in the examples herein is water meeting the pharmaceutical requirements, such as water for injection and purified water, and the oil is oil for injection. The preservatives described in the examples herein may also be referred to as antibacterial agents and play a role in inhibiting microbial growth and extending shelf life in formulations. The lubricants of the examples herein contain glidants and antiadherent. The sugars described in the examples herein may be powdered sugar or syrup, and the type of sugar is not limited to glucose. The fragrances described in the examples herein include, but are not limited to, flavors.

In some embodiments, the dosage of the pharmaceutical composition is at least 2 units, with 300 mg-400 mg per unit of the pharmaceutical composition.

In some embodiments, the dosage of the pharmaceutical composition is 2 units, with about 300 mg-400 mg per unit of the pharmaceutical composition. Further, the amount per unit of the pharmaceutical composition is selected from 300 mg, 320 mg, 340 mg, 350 mg, 380 mg, 400 mg, etc. In some embodiments, the administration frequency is once per three months. Further, considering the difference in the sustained-release effect of the drug among different individuals, the administration frequency is allowed to fluctuate within a reasonable range, for example, it may be once per 2.5-3.5 months.

In some embodiments, the mode of administration of the pharmaceutical composition is implantation. Further, in some embodiments, the implantation site is the abdominal cavity and arm.

In some embodiments, the dosage administered is at least 2 units of the pharmaceutical composition, with 300 mg-400 mg per unit of the pharmaceutical composition.

In some embodiments, the dosage administered is 2 units, with about 300 mg-400 mg per unit of the pharmaceutical composition. Further, the amount per unit of the pharmaceutical composition is selected from 300 mg, 320 mg, 340 mg, 350 mg, 380 mg, 400 mg, etc.

In some embodiments, the subject is a mammal.

In some embodiments, the subject is a rat or a human.

In some embodiments, the subject is an LE rat. Further, the dosage administered is at least 2 units, with 300 mg-400 mg per unit of the pharmaceutical composition. The pharmaceutical composition for administration includes at least 300 mg of naltrexone and at least 30 mg of risperidone.

In order to improve the sustained-release effect, the pharmaceutical composition may be prepared as a sustained-release implant.

In some of these embodiments, a method for preparing the sustained-release implant includes the steps of:
preparing naltrexone into naltrexone sustained-release microspheres;
preparing risperidone into risperidone sustained-release microspheres; and
weighing a required amount of risperidone sustained-release microspheres and naltrexone sustained-release microspheres according to a weight ratio, and adding pharmaceutically acceptable auxiliary materials to prepare an implant.

A mass ratio of naltrexone to risperidone in the implant is (5-30): 1.

In some of these embodiments, the pharmaceutical composition includes, by mass percentage: 37.5%-50% naltrexone, about 3.75%-5% risperidone, and 0.4%-0.6% magnesium stearate.

In some of these embodiments, the method for preparing the sustained-release implant refers to the technical solution in WO2022095203A, and the prepared sustained-release implant has technical effects consistent with those described in the patent, including: 1) sustained release in vitro for more than 12 weeks; 2) release following an approximately zero-order pattern with stable release rate; and 3) synchronized in vivo release rates of naltrexone sustained-release microspheres and risperidone sustained-release microspheres.

In some examples, three months after implantation, the sustained-release implant can still play an anti-relapse role in cannabis addiction.

The following are some specific examples.

For the experimental parameters not specified in the following specific examples, reference is preferably made to the guidelines given in the present document, and reference may also be made to experimental manuals in the art or other experimental methods known in the art, or to the experimental conditions recommended by the manufacturer.

The active pharmaceutical ingredients and reagents referred to in the specific examples below are either commercially available or can be prepared by a person skilled in the art according to known means.

### 1. Study of pharmaceutical composition preventing the relapse in patients with cannabis addiction

### Example 1

This example provides a method for preparing an implant containing an active pharmaceutical ingredient and products prepared by the method, including a naltrexone implant, a risperidone implant, and a naltrexone-risperidone compound implant.

### (1) Preparation of naltrexone implant

### ①Preparation of naltrexone microspheres

Naltrexone and poly (D, L-) lactide were dissolved in dichloromethane to form an oil phase. A continuous phase solution of water, surfactant polyvinyl alcohol, and osmotic agent sucrose was prepared. The oil phase was added dropwise to the continuous phase solution, stirred well to homogenize the volatile organic solvent, filtered, and dried under vacuum to obtain dry microspheres.

### ②Material mixing and pressing into tablets

The above-mentioned naltrexone microspheres and magnesium stearate were added into a hopper mixer in a prescribed amount to mix well, and then pressed to obtain uncoated tablets, with the strength of 150 mg/tablet.

### ③Coating and drying

The uncoated tablets were dip-coated with an ethyl acetate solution of poly (D, L-) lactide and then dried.

### ④Packaging

The tablets were filled into a penicillin vial according to the specified filling volume and sealed by pressing the stopper and rolling the cap.

### (2) Preparation of risperidone implant

### ①Preparation of risperidone microspheres

Sodium chloride was dissolved in purified water to form an internal water phase. Risperidone and poly (D, L-) lactide were dissolved in dichloromethane to form an oil phase. A continuous phase solution of water, surfactant polyvinyl alcohol, and osmotic agent sodium chloride was prepared. Then, the internal water phase was added to the oil phase to form a primary emulsion through ultrasonic emulsification. Finally, the primary emulsion was added dropwise to the continuous phase solution, stirred well to homogenize the volatile organic solvent, filtered, and dried under vacuum to obtain dry microspheres.

### ②Material mixing and pressing into tablets

The above-mentioned risperidone microspheres and magnesium stearate were added into a hopper mixer in a prescribed amount to mix well, and then pressed to obtain uncoated tablets, with the strength of 15mg/tablet.

### ③Coating and drying

The uncoated tablets were dip-coated with an ethyl acetate solution of poly (D, L-) lactide and then dried.

### ④Packaging

The tablets were filled into a penicillin vial according to the specified filling volume and sealed by pressing the stopper and rolling the cap.

### (3) Preparation of naltrexone-risperidone compound implant

### ①Preparation of naltrexone microspheres

Naltrexone and poly (D, L-) lactide were dissolved in dichloromethane to form an oil phase. A continuous phase solution of water, surfactant polyvinyl alcohol, and osmotic agent sucrose was prepared. The oil phase was added dropwise to the continuous phase solution, stirred well to homogenize the volatile organic solvent, filtered, and dried under vacuum to obtain dry microspheres.

②Sodium chloride was dissolved in purified water to form an internal water phase. Risperidone and poly (D, L-) lactide were dissolved in dichloromethane to form an oil phase. A continuous phase solution of water, surfactant polyvinyl alcohol, and osmotic agent sodium chloride was prepared. Then, the internal water phase was added to the oil phase to form a primary emulsion through ultrasonic emulsification. Finally, the primary emulsion was added dropwise to the continuous phase solution, stirred well to homogenize the volatile organic solvent, filtered, and dried under vacuum to obtain dry microspheres.

### ③Material mixing and pressing into tablets

The above-mentioned compound microspheres and magnesium stearate were added into a hopper mixer in a prescribed amount to mix well, and then pressed to obtain uncoated tablets, with the specification of naltrexone; risperidone being 150 mg: 15 mg/tablet.

### ④Coating and drying

The uncoated tablets were dip-coated with an ethyl acetate solution of poly (D, L-) lactide and then dried, with the weight of each naltrexone-risperidone implant tablet being 300 mg-400 mg.

### ⑤Packaging

The tablets were filled into a penicillin vial according to the specified filling volume and sealed by pressing the stopper and rolling the cap.

In examples 2-4 below, the effects of the pharmaceutical composition of the present application in the treatment of cannabis addiction and prevention of relapse were studied.

Subcutaneous implantation method of naltrexone sustained-release formulation was as follows: One day before implantation of a test article, two sides of the animal's back spine were shaved to expose the skin to facilitate the implantation of the naltrexone implant. After the animal was anesthetized with isoflurane, the local skin was disinfected with an alcohol cotton ball. A wound with a size of about 0.5 cm was cut with a surgical knife, and a puncture needle was pushed forward by about 3 cm-4 cm and moved left and right to ensure that it entered the cortex correctly, and then tablets with a corresponding dosage were injected. The injection site should be as far away from the spine as possible to avoid pain and injury to animals due to animal movement and tablet friction. After suturing, anti-inflammatory powder was sprinkled on the wound to ensure wound healing. Later, the wound surface was disinfected with iodophor every day until the wound was completely healed. Surgical instruments were sterilized in advance.

### Example 2

In this example, the anti-relapse effect of the pharmaceutical composition of the present application in an LE rat self-administration model was studied.

### 2.1. Drugs and reagents

WIN 55,212-2, off-white powder (131543-23-2, Shanghai Yuansi Standard Science and Technology Co., Ltd.);
Rimonabant, white solid (HY-14137, Shanghai Haoyuan ChemExpress Co., Ltd.);
Naltrexone-risperidone compound implant prepared in example 1;
Naltrexone implant prepared in example 1; and
Risperidone implant prepared in example 1.

In the following examples, the drugs used included solutions, and their preparation solvents and dosages were listed in Table 1.

**Table 1**

| Compound name | Drug preparation and solvent used |
|---|---|
| SK 1908 | None |
| Rimonabant | 1% castor oil, 1% anhydrous ethanol, and 18% sodium chloride injection |
| WIN 55,212-2 | 1% Tween 80 and 99% sodium chloride injection |

### 2.2. Experimental animals

SPF LE rats (Beijing Vital River Laboratory Animal Technology Co., Ltd.), weighing 220 g-250 g, were randomly grouped into 10 rats per group.

### 2.3. Establishment of self-administration model

Experimental instruments included: a rat self-administration system/16-channel and a rat self-administration operation cage with a dimension of 30.5 cm × 24.1 cm × 21 cm. The rat self-administration operation cage was placed in a ventilated and soundproof box, and two lever operating devices with 2.5 cm aperture were equipped on an operating face of each experimental cage as an "effective" lever and an "ineffective" lever, respectively. The spacing between the levers was 15 cm, and the height of the lever was 5 cm. An LED signal lamp was mounted above each lever. A white LED cage lamp with a diameter of 1 cm was mounted on the top of the operating face, and an infusion connection system includes an infusion pipeline, an infusion rotating shaft, and an infusion pump. The infusion pipeline was sheathed by an infusion spring connecting rod to protect the infusion pipeline from being bitten by an animal. The experimental cages were controlled by a computer.

### 2.4. Experimental method

### 2.4.1. Food training

On the third day of postoperative recovery, food was restricted to 12 g. From the fourth to the sixth day, food granule self-feeding training was performed at a fixed ratio of 1 (FR1) for 1 h per day to help learn the lever pressing behavior. Pressing the effective lever led to: (i) a cue light above the effective lever turning on for 10 s; (ii) a food granule appearing in a food trough; and (iii) the room light turning off for 20 s, and entering a time-out period. After completing the 20 s of time-out isolation, the room light turned on, and a new trial was started.

The other lever press was the ineffective lever press, not coupled with any consecutive events, but always recorded.

### 2.4.2. Self-administration training

7 days after postoperative recovery, (WIN55,212-2, WIN55,212-2 was cannabis injection with a concentration of 12.5 ug/kg/infusion, and the rat had a body weight of about 200 g) self-administration training was performed: rats were placed in a self-administration training box and connected to a self-administration injection system. Intensive training was performed using a fixed ratio of 1 (FR1, gradually increased to FR3) for 2 h per day with continuous training. In each self-administration process, the effective lever was activated, and the injection system performed automatic administration (intraperitoneal injection). Each injection was about 0.1 mL, with a duration of 4 s-6 s. Meanwhile, each injection would be accompanied by the effective lever light turning on for 10 s and the room light turning off for 20 s. The room light turned off to enter 20 s of the time-out period, during which the rats touched the effective lever without drug injection, but the effective lever pressing response number could be recorded. Data of activating the ineffective lever was recorded, but no drug injection was given. During the training, the computer automatically recorded the number of effective and ineffective lever presses and the number of injections. During the training, food was restricted to 12 g. Training success criteria were: the number of cannabis injections of the rats gradually increased and stabilized with the accumulation of training days, and the difference in the number of effective lever presses in the last 3 d stabilized at about 20%.

### 2.4.3. Self-administration regression training

After the establishment of the self-administration model, the rats received 2 h of environmental regression for each round of regression training. During the regression, only the spatial environment and lever during the training were presented, without other clues such as lights and pump sounds as well as drug injections. The computer automatically recorded the number of behavioral responses of rats, and the rats reached the regression criteria (i.e., the total number of effective lever presses decreased to 20% of the total number of effective lever presses on the first day of the regression training or less than 10 times).

### 2.4.4. Grouping and administration

After completing self-administration training and regression, the rats were randomly divided into a naltrexone-risperidone compound implant group, a naltrexone implant group, and a risperidone implant group according to the number of injections and the number of effective lever presses. The naltrexone-risperidone compound implant, naltrexone implant, and risperidone implant were implanted subcutaneously. In the control group and rimonabant group, sham operations were performed. After 3 days of postoperative care, a corresponding relapse test was started on the fourth day.

### 2.4.5. Effect of compound on environmental cue-induced relapse behavior

In the rimonabant group, rats were administered the rimonabant injection at a dosage of 2 mg/kg 15 min before the test. In the control group, rats were administered normal saline 15 min before the test. In the naltrexone-risperidone compound implant group, naltrexone implant group, and risperidone implant group, there would be no administration. Then, animals were subjected to 2 h of environmental cue-induced relapse test, and the cues were consistent with those of self-administration training, including levers, cage light, and syringe pump sound, but no drug in the syringe pump. The number of injections and effective lever pressing data were recorded by the computer.

### 2.4.6. Regression training

The rats received 2 h of environmental regression again. During the regression, only the spatial environment during the self-administration training was presented, without other clues such as lights and pump sounds as well as drug injections. The computer automatically recorded the number of behavioral responses of rats, and the regression was continued until reaching the criteria.

### 2.4.7. Effect of compound on drug (WIN55,212-2) and environmental cue-induced relapse behavior

In the rimonabant group, rats were administered the rimonabant injection (2 mg/kg, ip) 15 min before the test through intraperitoneal injection at a dose of 2 mg per kilogram of body weight. In the control group, rats were administered normal saline 15 min before the test. In the implant administration groups, there would be no administration. All rats were administered cannabis (WIN55,212-2 injection 0.3 mg/kg, ip) through intraperitoneal injection at a dose of 0.5 mg per kilogram of body weight, and then a 2-h drug and environmental cue-induced relapse test was immediately performed. The cues were consistent with those of self-administration training, including levers and syringe pump sound, but no drug in the syringe pump. The number of injections and effective lever pressing data were recorded by the computer.

### 2.5. Experimental results

### 2.5.1. Results of effect of compound on environmental cue-induced relapse behavior

In the environmental cue-induced relapse test, the control group showed significant relapse behaviors, as evidenced by a significant increase in the number of effective lever presses compared with the last regression. In the naltrexone implant group and the naltrexone-risperidone compound implant group, the cue-induced relapse behavior was significantly reduced, as evidenced by a significant decrease in the number of effective lever presses compared with the control group (Table 2). The reference compound rimonabant group showed no significant inhibition effect on the relapse test. There was no significant difference between groups in the number of ineffective lever presses.

**Table 2 Effect of compound on environmental cue-induced relapse behavior (mean ± SD, n = 10).**

| Group | Dosage | Number of effective lever presses | | Number of ineffective lever presses | |
|---|---|---|---|---|---|
| | | Last-Ext | Cue priming | Last-Ext | Cue priming |
| Vehicle | 0 | 11.27±2.14 | 83.00±23.81**** | 5.64±2.04 | 7.36±1.71 |
| Rimonabant | 2 mg/kg | 6.80±1.62 | 57.70±10.27 | 3.10±0.86 | 12.10±3.33 |
| Naltrexone-risp eridone compound implant group | 4 tablets/rat | 12.75±2.73 | 27.75±3.77^{##} | 2.00±0.60 | 3.33±0.94 |
| Risperidone implant group | 4 tablets/rat | 11.65±2.35 | 65.78±7.65 | 4.01±0.75 | 8.15±1.76 |
| Naltrexone implant group | 4 tablets/rat | 10.51±1.91 | 45.31±6.78^{#} | 3.11±0.89 | 6.05±1.02 |

In Table 2, data were expressed as Mean ± SEM, and two-way repeated analysis of variance was adopted (compared with the last-ext-Vehicle group: ****p<0.0001; compared with the Cue-Vehicle group: ^{#}p<0.05, and ^{##}p<0.01).

### 2.5.2. Effect of compound on drug and environmental cue-induced relapse behavior

In the drug and environmental cue-induced relapse test, the control group showed significant relapse behaviors, as evidenced by a significant increase in the number of effective lever presses compared with the last regression. However, in the naltrexone implant group and the naltrexone-risperidone compound implant group, the cue and drug-induced relapse behavior was significantly reduced, as evidenced by a significant decrease in the number of effective lever presses compared with the control group (Table 3). The reference compound rimonabant group showed no significant inhibition effect on the relapse test.

**Table 3 Effect of compound on drug and environmental cue-induced relapse behavior (mean ± SD, n = 10)**

| Group | Dosage | Number of effective lever presses | | Number of ineffective lever presses | |
|---|---|---|---|---|---|
| | | Re-Ext | Cue + drug priming | Re-Ext | Cue + drug priming |
| Solvent control group | - | 13.27±2.20 | 48.91±12.03* | 5.73±1.2 | 1.50±0.54 |
| Rimonabant | 2 mg/kg | 10.30±2.72 | 63.50±16.15 | 5.20±3.0 | 8.10±2.08 |
| Naltrexone-risper idone compound implant group | 4 tablets/rat | 7.750±1.18 | 18.25±4.52## | 1.50±0.54 | 1.75±0.46 |
| Risperidone implant group | 4 tablets/rat | 9.99±2.65 | 40.24±11.65 | 5.40±2.1 | 5.99±0.47 |
| Naltrexone implant group | 4 tablets/rat | 8.97±2.34 | 29.12±8.69# | 3.64±0.66 | 2.41±0.52 |

Data were expressed as Mean ± SEM, and two-way repeated analysis of variance was adopted (compared with the re-ext-Vehicle group: *p<0.05; compared with the Cue+Drug-Vehicle group: #p<0.05, and ##p<0.01).

In this example, LE female rats could form stable self-administration of WIN 55,212-2, indicating successful model establishment. After completing the self-administration training and regression, the rats were randomly divided into different groups, and there was no significant difference in the training and regression stages among the control group, rimonabant group, naltrexone implant group, risperidone implant group, and naltrexone-risperidone compound implant group. In the environment-induced relapse test, the control group showed significant relapse behavior, while in the naltrexone implant group and the naltrexone-risperidone compound implant group, the cue-induced relapse behavior could be significantly reduced. In the drug and environmental cue-induced relapse test, the control group showed significant relapse behavior, while in the naltrexone implant group and the naltrexone-risperidone compound implant group, the cue and drug-induced relapse behavior could be significantly reduced. The positive drug rimonabant showed no significant inhibition effect on the two relapse tests. It can be seen that the naltrexone-risperidone compound implant has good efficacy in preventing the relapse into cannabis use and has better efficacy than a single agent.

### Example 3

In this example, the effect of the pharmaceutical composition of the present application on addiction formation of LE rat self-administration was studied.

### 3.1 Grouping and administration

Animals were randomly divided into model control group, rimonabant group, naltrexone-risperidone compound implant group, naltrexone implant group, and risperidone implant group. On the day of venous catheterization, the naltrexone-risperidone compound implant, naltrexone implant, and risperidone implant were implanted subcutaneously. In the control group and rimonabant group, sham operations were performed. After 3 days of postoperative care, addiction formation training was started on the fourth day.

### 3.2 Effect of drug on the establishment of intravenous self-administration model

In the control group and rimonabant group, rats were administered the corresponding compound 15 min before the daily training. The rimonabant was at a dosage of 2 mg/kg, i.p. In the control group, rats were administered normal saline, and in the implant administration groups, there would be no administration. The training operation was the same as the self-administration training in example 1. During the training, the computer automatically recorded the number of effective and ineffective lever presses and the number of injections. During the training, food was restricted to 12 g. Training success criteria were: the number of cannabis injections of the rats gradually increased and stabilized with the accumulation of training days, and the difference in the number of effective lever presses in the last 3 d stabilized at about 20%.

### 3.3 Effect of drug on regression of drug-seeking behavior

After the establishment of the self-administration model, the rats received 2 h of environmental regression for each round of regression training. During the regression, only the spatial environment and lever during the training were presented, without other clues such as lights and pump sounds as well as drug injections. The computer automatically recorded the number of behavioral responses of rats, and the rats reached the regression criteria (i.e., the total number of effective lever presses decreased to 20% of the total number of effective lever presses on the first day of the regression training or less than 10 times).

### 3.4 Experimental results

The number of effective lever presses at the time of regression and the number of days to reach stable regression were recorded. The results are shown in Table 4. According to Table 4, administration of rimonabant, naltrexone-risperidone compound implant, and naltrexone implant significantly inhibited the increase in the number of effective lever presses caused by addiction formation of win55,212-2 (cannabis). Except for the risperidone administration group, other administration groups had significant difference compared with the control group, in which the naltrexone-risperidone compound implant group had the strongest inhibition effect. This indicated that the combination of naltrexone and risperidone had a synergistic effect compared with naltrexone or risperidone alone.

In the regression process, the order of the groups that reached the regression criteria was the naltrexone-risperidone compound implant group, naltrexone group and rimonabant group equivalent, risperidone group, and normal saline control group. The results showed that each administration group had a certain inhibition effect on the regression process of cannabis addiction, and the naltrexone-risperidone compound implant group had the most significant inhibition effect on the regression of cannabis addiction, which was better than the combination administration group and single drug group.

**Table 4 WIN55,212-2 self-administration training and regression of female LE rats (mean ± SD, n = 10)**

| Group | Dosage | Addiction formation (26 days) | | Regression (13 sessions) | |
|---|---|---|---|---|---|
| | | Number of effective lever presses | Number of ineffective lever presses | Number of effective lever presses | First stabilization (days) |
| Vehicle | - | 86.74±4.00 | 9.29±0.72 | 17.60±5.73 | 13 |
| Rimonabant | 2 mg/kg | 45.70±8.21* | 9.49±1.05 | 9.27±2.21 | 7 |
| Naltrexone-risperidone compound implant group | 4 tablets/rat | 20.65±3.10** * | 9.50±0.94 | 5.96±2.17 | 3 |
| Risperidone implant group | 4 tablets/rat | 59.14±8.11 | 9.75±0.71 | 10.71±1.01 | 11 |
| Naltrexone implant group | 4 tablets/rat | 40.22±6.32* | 8.97±1.01 | 8.30±1.92 | 7 |

Data were expressed as Mean ± SEM, and two-way repeated analysis of variance was adopted (compared with the Vehicle group: *p<0.05, **p<0.01, and ***p<0.001).

### 3.5. Conclusion

### 3.5.1 Role of rimonabant in different stages of cannabis addiction

According to the experimental data, rimonabant has a significant inhibition effect on the formation of cannabis addiction and may accelerate the regression process and reduce the regression days. It also has an inhibition effect on the cue priming and cue + drug priming, but the effect is not significant. Rimonabant is suggested to prevent cannabis addiction.

The behavioral process of drug addiction is divided into three stages: (1) formation; (2) withdrawal (cessation of drug in a living environment) or regression (cessation of drug in a training environment; specific cue regression, environmental cue regression, and specific + environmental cue regression); (3) relapse (triggered by cues or low-dose drug exposure). In the formation of cannabis addiction, cannabinoids enter the body to increase dopamine levels by activating CB1 receptors within the ventral tegmental area, thereby inducing their enhancing properties. Subsequently, compensatory adaptation has also occurred in some non-CB1 receptor systems, including dopamine receptor systems, 5-HT receptor systems, opioid receptor systems, and GABA receptor systems. Rimonabant is a CB1 receptor inhibitor, which is considered a positive drug for a cannabis addiction model. The blocking effect of rimonabant on CB1 receptors is more effective in the early stage of the formation of cannabis addiction, and in the relapse stage of cannabis addiction (including cue priming and cue + drug priming), the non-CB1 receptor system plays a very important role, and the blocking effect of CB1 receptors is limited, so the effect of rimonabant is limited.

### 3.5.2. Roles of naltrexone-risperidone in different stages of cannabis addiction

It can be seen from the experimental data that when the implantation amount of the naltrexone-risperidone compound implant is greater than 2 tablets, i.e., 300 mg of naltrexone: 30 mg of risperidone, compared with the model group, the compound implant can significantly inhibit the formation and relapse of cannabis addiction in cannabis addiction formation, regression, cue priming, and cue + drug priming, and can significantly accelerate the stable formation of regression. Therefore, the naltrexone-risperidone implant can not only prevent the formation of cannabis addiction and accelerate the withdrawal of cannabis, but also effectively prevent relapse into cannabis use.

### Example 4

In this example, the effects of different dosages of the pharmaceutical composition on the self-administration relapse behavior and plasma concentration results were studied.

### 4.1. Grouping and administration

The training operation was the same as in example 1. After completing the self-administration training and regression, the rats were randomly divided into a control group and low-dosage, medium-dosage, and high-dosage naltrexone-risperidone compound implant groups according to the number of injections and the number of effective lever presses. Low-dosage, medium-dosage, and high-dosage naltrexone-risperidone compound implants were implanted subcutaneously. In the control group, a sham operation was performed. After 3 days of postoperative care, a corresponding relapse test was started on the fourth day. At the end of the relapse test, immediately after the end of the experiment, blood was immediately acquired from the heart of the animals and centrifuged for plasma, and the drug contents of naltrexone, 6-β-naltrexol, risperidone, and 9-OH-risperidone in plasma were determined.

### 4.2. Effect of drug on environmental cue-induced relapse behavior

In the control group, rats were administered normal saline 15 min before the test, and in the implant groups, there would be no administration. Then, animals were subjected to 2 h of environmental cue-induced relapse test, and the cues were consistent with those of self-administration training, including levers, cage light, and syringe pump sound, but no drug in the syringe pump. The number of injections and effective lever pressing data were recorded by the computer.

### 4.3. Regression training

The rats received 2 h of environmental regression again. During the regression, only the spatial environment during the self-administration training was presented, without other clues such as lights and pump sounds as well as drug injections. The computer automatically recorded the number of behavioral responses of rats, and the regression was continued until reaching the criteria.

### 4.4. Effect of drug on drug and environmental cue-induced relapse behavior

In the control group, rats were administered normal saline 15 min before the test, and in the implant groups, there would be no administration. All rats were administered WIN55,212-2 (0.3 mg/kg, i.p) through intraperitoneal administration, and then a 2-h drug and environmental cue-induced relapse test was immediately performed. The cues were consistent with those of self-administration training, including levers and syringe pump sound, but no drug in the syringe pump. The number of injections and effective lever pressing data were recorded by the computer.

### 4.5. Experimental results

The results of cue priming and cue + drug priming by the compound naltrexone-risperidone groups of different dosages are shown in the following table (Table 5). The experimental results show that with the increase of the dosage (number of tablets) of the implants, the plasma concentration in rats increases accordingly, and the specific results are shown in the following table. In a win55,212-2-induced addiction model, the compound implant had a significant inhibition effect on the cue priming and cue + drug priming when implanted with 4 and 2 tablets, and had a slight inhibition effect when implanted with 1 tablet, but there was no significant difference. The inhibition effect of the implant on the relapse was dosage-dependent.

That is, 2 tablets of the naltrexone-risperidone compound implant began to take effect, each tablet was 150 mg/15mg, and the sustained-release time was 3 months.

**Table 5 Effects of different dosages of drugs on self-administration relapse behavior and plasma concentration results (mean ± SD, n = 10)**

| Group | | Vehicle | Compound-H | Compound-M | Compound-L |
|---|---|---|---|---|---|
| Dosage | | - | 4 tablets/rat | 2 tablets/rat | 1 tablet/rat |
| Number of effective lever presses (priming) | Last-Ext | 15.11±2.15 | 11.24±2.35 | 12.33±3.11 | 12.42±2.54 |
| | Cue | 81.01±20.33**** | 24.63±2.65## | 42.31±6.02# | 63.65±5.96 |
| | Last-Ext | 11.21±1.96 | 10.11±2.31 | 13.87±3.12 | 13.55±2.65 |
| | Cue+Drug | 50.22±11.11** | 19.22±4.21^{##} | 30.99±3.65^{#} | 43.15±2.34 |
| Plasma concentrat ion ng/ml | Naltrexone | - | 75.12±20.60 | 30.25±10.11 | 13.52±3.80 |
| | 6β-naltrexol | - | 2.47±0.84 | 1.02±0.41 | 0.35±0.15 |
| | Risperidone | - | 52.14±14.26 | 20.12±6.98 | 4.29±1.87 |
| | 9-hydroxyrispe ridone | - | 37.46±8.62 | 10.11±3.55 | 2.67±1.48 |

In Table 5, data were expressed as Mean ± SEM, and two-way repeated analysis of variance was adopted (compared with the Last-ext-Vehicle group: **p<0.01, and ****p<0.0001; compared with the Cue-Vehicle group and the Cue+Drug-Vehicle group: #p<0.05, and ##p<0.01).

### 2. Study of therapeutic effect of pharmaceutical composition on adverse symptoms caused by cannabis addiction

This example was a study on the effectiveness and safety of the naltrexone-risperidone implant for anti-relapse therapy after detoxification in cannabis-dependent patients and related to the improvement effect on mental symptoms by the naltrexone-risperidone implant for anti-relapse therapy after detoxification in cannabis-dependent patients.

### 1. Experimental design

This study was an investigator-initiated, single-center, randomized, open, blank-controlled clinical trial. 100 cannabis-dependent patients were selected.

Subjects participating in the trial must meet all of the following criteria to enter the study:
(1) voluntarily participate in the clinical study; fully understand and be informed about the study and sign the informed consent form; be willing to follow and have the ability to complete all trial procedures;
(2) be aged 18 years or older at the time of signing the informed consent form;
(3) meet the DSM-5 diagnostic criteria for cannabis use disorder and have completed detoxification therapy;
(4) have a negative urine cannabis qualitative test (TLC);
(5) female subjects must have evidence of postmenopausal status, or premenopausal female subjects must have a negative serum pregnancy test result before administration. Eligible subjects of childbearing age (males and females) must agree to take effective contraceptive measures (hormonal or barrier methods or abstinence) with their partners during the trial.

Subjects participating in the trial cannot be included if they meet any of the following criteria:
(1) the investigator determines that participation in this study is not in conformity with the rights and interests of the subject, or there are any other circumstances that would prevent the subject from safely participating in the study;
(2) pregnant women and women of childbearing age with a positive pregnancy test, or breastfeeding women, including women of childbearing age who plan to become pregnant during the study. Note: Women of childbearing age refer to women who are capable of becoming pregnant. The following criteria must be met, regardless of their sexual orientation or whether they have had tubal ligation: 1) have not undergone hysterectomy or bilateral oophorectomy; or 2) have not experienced natural menopause for more than 12 consecutive months (i.e., menstruation occurred at any time during the previous 12 months);
(3) a subject with significant hepatic insufficiency (e.g., AST or ALT more than twice the upper limit of normal), hepatic failure [including, but not limited to: ascites, prolonged prothrombin time (PT), international normalized ratio (INR) ≥1.7, esophageal varices disease], or the liver and gallbladder B-scan ultrasonography results that significantly affect the determination of the effectiveness and safety of the study drug;
(4) a subject with clinically uncontrolled active infectious diseases, such as active phase of hepatitis B [positive for hepatitis B surface antigen (HBsAg) and hepatitis B virus (HBV) deoxyribonucleic acid (DNA) copies >1000 IU/ml], active phase of hepatitis C [positive for hepatitis C virus antibody and positive for hepatitis C virus (HCV)-ribonucleic acid (RNA)], etc.;
(5) a subject with a history of congenital hemorrhagic disease (e.g., hemophilia), any clinically significant active hemorrhage, platelet function abnormalities, coagulation function tests with prothrombin time (PT) exceeding the upper limit of normal by more than 3 seconds (sec), or platelet count < 50× 109/L;
(6) as determined by the investigator, the subject has any severe/uncontrollable systemic diseases (e.g., respiratory, cardiovascular, digestive, nervous, hematologic, urogenital, reproductive and endocrine system diseases), mental diseases (e.g., major depressive disorder, schizophrenia, and bipolar disorder), or other significant diseases that the investigator believes would interfere with the provision of informed consent, pose safety risks for the participant, complicate the interpretation of outcome data, or otherwise affect the study objectives;
(7) a subject who may require hospitalization or surgery during the study, including planned elective surgery or non-deferrable hospitalization;
(8) a subject currently (within one year before randomization/administration) diagnosed with substance use disorders other than cannabis, such as benzodiazepines, opioids, or cocaine;
(9) a subject who used anti-relapse drugs (e.g., naltrexone, risperidone, etc.) within 30 days before randomization/administration;
(10) a subject currently receiving treatment for substance use disorders such as opioids and alcohol or receiving opioids within 7 days before randomization/administration. Opioid treatment may be required during the study, or on the day of randomization/administration, positive for urine opioids, cannabis, and other drugs test or positive for naloxone challenge test may be required;
(11) a subject who is allergic to the study drug, auxiliary materials thereof (polylactic acid and magnesium stearate), and local anesthetics;
(12) a subject who is participating in any study drug or device study or has used any study drug or device within 30 days before randomization/administration;
(13) a subject with skin infection at the implantation site or systemic skin disease that affects the effectiveness and safety assessment of the study drug;
(14) a subject with clinical or laboratory evidence of human immunodeficiency virus (HIV) or syphilis carrier/infection.

Subjects who have been enrolled and, during the study, are found to be unsuitable for continuing the study, may be decided by the investigator to withdraw from the study, including:
(1) in the clinical trial, a subject experienced some comorbidities and complications or disease deterioration, and the investigator determines that the subject is not suitable to continue the trial;
(2) major protocol deviation, after randomization/administration, it is found that the subject does not meet the inclusion criteria of the study protocol, or the subject has poor compliance, does not follow the requirements of the study protocol or medical advice, and uses other treatments without authorization, affecting the assessment of efficacy and safety, or the investigator determines that continued participation in the study may pose an unacceptable risk to the subject's health;
(3) the investigator considers that continued treatment is not beneficial to the patient (e.g., pregnancy);
(4) a subject experiences an adverse event (AE) or a serious adverse event (SAE), and the investigator determines that the subject is not suitable to continue the trial;
(5) lack of effectiveness. the investigator determines that the subject does not benefit from the study treatment and that continued participation in the study may pose an unacceptable risk to the subject;
(6) other factors not involved above shall be noted.

Subjects who are unwilling to continue participating in the clinical study (the subjects have the right to withdraw from the study at any stage according to the informed consent form), including:
(1) a subject who does not explicitly request to withdraw from the study but are lost to follow-up (or "drop-out") as they no longer accept testing;
(2) a subject who is unable to continue participating in the clinical study for other reasons;
(3) a subject who is lost to follow-up without explanation. Lost to follow-up is defined as loss of contact for the subject. The investigator should understand and record the reasons for withdrawal or drop-out as far as possible. For example: the subject feels intolerable due to certain adverse reactions.

For patients who discontinue or withdraw from the study, the naltrexone implant or placebo is removed. The investigator must fill out the reason for withdrawal in the case report form, contact the patient if possible, complete as many assessment items as possible, fill out the treatment follow-up record form, and record the last follow-up time if possible. Those who withdraw from the study due to AEs and are finally determined to be related to the study drug upon follow-up must be recorded in the case report form and notified to the sponsor. Regardless of the reason, for withdrawal cases, the case report form should be kept. The final visit should be completed, and the last test result is taken as the final result. A full data analysis on efficacy and adverse reactions should be performed. All existing study-related toxicities and SAEs must be followed up for remission at the time of withdrawal from the study, unless, in the opinion of the investigator, the condition is unlikely to be resolved due to the patient's disease itself. For patients who withdraw from the study in advance, no replacement will be given. For patients who withdraw from the study in advance, if there is a continuing need for alcohol withdrawal treatment (referral or hospitalization), necessary medical support should be provided, and their safety should continue to be followed up. For subjects who withdraw from the study in advance but refuse to remove the naltrexone implant or placebo, safety follow-ups (telephone follow-ups for AEs and concomitant medication information) should be conducted at each subsequent scheduled visit, unless the subject explicitly states that they no longer accept any follow-ups.

Subjects participating in the trial, after signing the informed consent form and passing the inclusion/exclusion criteria screening, would be randomized in a 2:1 ratio to a drug treatment group (treatment with the naltrexone-risperidone implant) or to a blank group.

Subjects were randomly screened in the detoxification center from Day-28 to Day-1 before administration. On Day 0, the subjects were implanted with the naltrexone-risperidone implant (naltrexone: risperidone = 150 mg: 15mg), and in the blank group, no therapeutic drugs were given. The patients were followed up once a week for 14 weeks after administration.

### 1. Efficacy indicators

### 1.1 Primary efficacy indicators

The primary efficacy indicators included the percentage of time (weeks) without cannabis use, specifically recording whether cannabis was used every week and determining the number of weeks without cannabis use. The "number of weeks without drug use" was divided by the "number of weeks at risk for drug use", and the above days were calculated until the efficacy observation was stopped. The "number of weeks at risk for drug use" is defined as the number of weeks that a subject receives the efficacy observation in the study. The "number of weeks without drug use" is defined as negative urine tests at weekly follow-ups.

### 1.2 Secondary efficacy indicators

The secondary efficacy indicators included (1) time of patient drop-out (calculated from the first administration); (2) cannabis craving questionnaire (MCQ): craving scale scores from baseline to pre-specified weekly visits; (3) incidence of subjects with recurrent physiologic drug dependence during 14 weeks of treatment; (4) brief psychiatric rating scale (BPRS): BPRS from baseline to pre-specified weekly visits.

The time of patient drop-out (calculated from first administration) refers to the time from randomization/administration to loss to follow-up/end of trial.

The MCQ refers to: cannabis craving scores weekly after randomization/administration. Items were scored according to a Likert-type scale from 1 (strong disagreement) to 7 (strong agreement). The factor score for each participant was obtained by summing 3 items in each factor scale, resulting in a score ranging from 3 to 21. A four-factor scale included: 1) compulsivity, uncontrolled use of cannabis; 2) emotionality, smoking cannabis to alleviate withdrawal or negative emotions; 3) expectancy, expectancy for positive results of cannabis smoking; 4) purpose, intent, and plan to use cannabis for positive results. The highest score was 84, and the lowest score was 12. The higher the score, the more severe cannabis craving symptoms recognized by the subject.

The incidence of recurrent physiologic drug dependence among subjects during the 14-week treatment period is defined as: the proportion of relapse patients from randomization/administration to loss to follow-up/end of trial.

The BPRS refers to: psychiatric scores assessed weekly following randomization/administration. The BPRS is a psychiatric rating scale that enables clinicians or researchers to measure psychiatric symptoms of patients, such as depression, anxiety, hallucinations, and other abnormal behaviors. It particularly focuses on psychiatric symptoms and live conversations within the last week. The BPRS is a 7-point rating scale that assesses the severity, frequency, and duration of symptoms, as well as the impact of symptoms on relevant functions. The ratings are categorized as follows: (1) asymptomatic; (2) suspicious or very mild, with some evidence present but the clinical significance is not certain; (3) mild, with mild symptoms but potential clinical significance; (4) moderate; (5) moderately severe; (6) severe; and (7) extremely severe. The total score (168 points) reflects the severity of the disease. A higher total score indicates a more severe condition. Change in total score before and after treatment reflect the efficacy of the treatment, with a greater difference suggesting better treatment outcome. Generally, a study inclusion criterion may be set at a score greater than 35.

### 2. Safety assessment indicators

AEs and SAEs (including local AEs and SAEs at the implantation site), as well as the occurrence of abnormalities in vital signs, physical examinations, 12-lead or higher electrocardiograms, and laboratory safety data (blood routine examination, urine routine examination, clinical biochemistry, and coagulation tests) before and after treatment were included.

### 3. Test drug

The test drug was the naltrexone-risperidone implant prepared in example 1, with the strength of 150 mg: 15 mg/tablet.

The administration method and dosage were abdominal subcutaneous implantation and single administration.

4. During the weekly screening period (from Day-28 to Day-1), the subjects participating in the trial would undergo a screening visit (visit 1), and the qualified subjects would be randomized at visit 2 (D0). Subjects enrolled in the trial group would receive naltrexone-risperidone implant treatment. The subjects received a single abdominal subcutaneous implantation for administration on the day of randomization (D0, visit 2). In the control group, no medication was administered. Patients were required to return to the study center once a week for follow-up to 14 weeks after randomization/administration. There were a total of 15 follow-ups.

The calculation method of the sample size was as follows: Assuming that the relapse rate decreased by 20% after drug administration, with a natural compliance rate of 40% and a compliance rate of 70% in the trial group over a 14-week period, the statistical calculation indicated that a sample size of 100 cases was required to meet the statistical requirements.

The calculation method of an analysis set was as follows:
A randomized analysis set included all randomized subjects according to the principle of an intention-to-treat analysis (ITT).

A per-protocol set (PPS) included subjects who completed the 14-week follow-ups after randomization/administration in all randomized analysis sets without serious violation of study protocol. As a subset of the randomized analysis set, it would be labeled in the randomized analysis set.

A safety set (SS) included all subjects who were randomized, received study drug treatment, and had at least one safety assessment.

Demography and baseline characteristics were analyzed using the randomized analysis set, and the efficacy was analyzed using the randomized analysis set. PPS-based analyses could provide complementary arguments, and safety analysis was based on the SS.

The efficacy analysis method was as follows: The efficacy analysis was based on the randomized analysis set, and PPS-based efficacy analysis results could provide complementary arguments.

The safety analysis method was as follows: The safety was analyzed based on the SS. The AEs were encoded using the terms in the medical dictionary for regulatory activities (MedDRA) (latest version when the analysis was performed), and descriptive statistical analysis was performed according to the system organ class (SOC)/preferred term (PT). The overall incidence of AEs, adverse reactions (i.e., AEs determined as not "definitely unrelated" in terms of relevance), AEs occurring during treatment, important AEs, and serious AEs, and the incidence of differentiated SOC/PT were calculated. According to the SOC/PT and severity, the number of individuals and occurrences of AEs and adverse reactions was counted. AEs, adverse reactions, AEs occurring during treatment, important AEs, and serious AEs were listed in detail.

For the laboratory examination, a normal/abnormal conversion cross table before and after administration was used to describe the change in results before and after administration. The laboratory examination results were listed in detail.

For the electrocardiogram examination, a normal/abnormal conversion cross table before and after administration was used to describe the change in results before and after administration. The measured values of QTc interval were statistically described as >450 ms, >480 ms, and >500 ms, with increases from baseline of >30 ms and >60 ms.

The results of electrocardiogram examination and liver and gallbladder B-scan ultrasonography examination were listed in detail.

Results of vital signs and physical examination indicators at each visit were analyzed and listed in detail. See general principles for analytical methods.

The flowchart of the trial was shown in Fig. 1. In Fig. 1, the patient should sign the informed consent form before starting any study-related procedures. Demographic information includes: gender, age, nationality, height, weight, body mass index, allergy history, drug abuse history, smoking history, alcohol abuse history, and blood donation history. Vital signs include: body temperature (forehead temperature), sitting blood pressure, heart rate, and respiratory rate. The sitting blood pressure and heart rate shall be measured after resting for 5 min. A comprehensive physical examination is conducted by organ and system, including: generally, head and neck, lymph nodes, skin, chest, abdomen, musculoskeletal system (including limbs and spine), and nervous system. Before randomization/administration, fasting blood and urine samples for laboratory testing should be acquired on the morning of the visit, including urine and/or blood samples for serum pregnancy tests for women with fertility potential.

Laboratory examinations include: 1) blood routine examination: including hemoglobin (Hb), hematocrit (HCT), mean corpuscular volume (MCV), red blood cell count (RBC), white blood cell count (WBC), white blood cell differential count (including neutrophils, lymphocytes, monocytes, basophils, and eosinophils), and platelets (PLT);
2) urine routine examination: including pH, urine protein (U-PRO), urine white blood cells (U-WBC), urine red blood cells (U-RBC), urine glucose (U-GLU), urine occult blood, and urine ketone (U-KET);
3) serum biochemistry: including aspartate aminotransferase (AST), alanine aminotransferase (ALT), alkaline phosphatase (ALP), lactate dehydrogenase (LDH), gamma-glutamyl transferase (GGT), total bilirubin (TBIL), direct bilirubin (DBIL), albumin (ALB), total protein (TP), triglyceride (TG), high-density lipoprotein cholesterol (HDL-C); serum creatinine (sCr), urea or blood urea nitrogen (BUN), uric acid (UA), potassium (K), sodium (Na), chloride (Cl), calcium (Ca), phosphorus (P), magnesium (Mg), and glucose (GLU);
4) pregnancy test (only for women of childbearing age): the serum pregnancy tests are conducted only during the screening period and visit 9, and the serum pregnancy test for women of childbearing age must be negative. Urine pregnancy tests may be performed as needed during the study treatment. If the urine pregnancy result is positive, a serum pregnancy test is required for confirmation. If confirmed to be positive, the subject should be withdrawn from the study, and the investigator will continue to follow up with the subject until the end of the pregnancy;
5) serum virology examination: including HIV antibody, HCV antibody, HBsAg, hepatitis B core antibody (HBcAb), HBV DNA, HCV-RNA, and syphilis-specific antibody (Anti-TP). The serum virology examination is only performed during the screening period;
6) coagulation function: including PT, INR (performed only during the screening period), activated partial thromboplastin time (APTT), thrombin time (TT), and fibrinogen (FIB);
7) 12-lead or higher electrocardiogram: including heart rate, ventricular rate, PR interval, QT/QTc interval, and QRS complex morphology after at least 5 minutes in the supine position;
8) liver and gallbladder B-scan ultrasonography: before randomization/administration, the test will be performed on the morning of the visit and when the subject is fasting, only at visit 1 and visit 14;
9) abdominal subcutaneous implantation of the naltrexone-risperidone implant is performed. After administration, the patient is kept in hospital for observation for 2 hours. On the third day after operation, the dressing at the wound site is changed by the patient;
10) for the subjects who withdraw from the study in advance, the withdrawal visit shall be conducted within 2 weeks after confirmation of withdrawal from the study, and the visit content shall be the same as the study completion visit.

The results of the study in this example indicate that the naltrexone-risperidone implant may effectively reduce the relapse rate among patients. During the visits at week 4 and subsequent weeks, the percentage of positive urine test results for patients receiving the implant was lower than that of the placebo group, suggesting that the naltrexone-risperidone implant could effectively prevent the relapse in patients with cannabis addiction. From the results of the MCQ, it can be observed that the naltrexone-risperidone implant could significantly reduce the patients' cravings for cannabis. Furthermore, according to the results of the BPRS, the naltrexone-risperidone implant appears to significantly improve the psychiatric symptoms of patients addicted to cannabis , particularly alleviating symptoms such as anxiety, depression, and hallucinations that are caused by cannabis addiction.

## Claims

1. A pharmaceutical composition for use in preventing relapse into cannabis use, wherein the pharmaceutical composition comprises naltrexone and risperidone as an active pharmaceutical ingredient.

2. The pharmaceutical composition for use according to claim 1, wherein a mass ratio of naltrexone to risperidone is (5-30): 1.

3. The pharmaceutical composition for use according to claim 2, wherein the mass ratio of naltrexone to risperidone is (8-15): 1.

4. The pharmaceutical composition for use according to any one of claims 1 to 3, further comprising a pharmaceutically acceptable carrier.

5. The pharmaceutical composition for use according to any one of claims 1 to 4, wherein the pharmaceutical composition is used together with pharmaceutically acceptable auxiliary materials to prepare a clinically acceptable formulation.

6. The pharmaceutical composition for use according to claim 5, wherein the formulation is selected from any of the following groups or a suitable combination thereof:
1) tablets, capsules, granules, powders, dispersible powders, suspensions, or solutions; and
2) oral liquids, buccal agents, injections, suppositories, sprays, drops, or patche

7. The pharmaceutical composition for use according to claim 6, wherein the formulation is in the form of an implant.

8. The pharmaceutical composition for use according to any one of claims 1 to 7, wherein a subject is a mammal.

9. The pharmaceutical composition for use according to claim 8, wherein the subject is a human or a rat.

10. The pharmaceutical composition for use according to any one of claims 1 to 9 in the treatment of adverse symptoms caused by cannabis addiction.

11. The pharmaceutical composition for use according to claim 10, wherein the adverse symptoms caused by cannabis addiction comprise psychiatric symptoms.

12. The pharmaceutical composition for use according to claim 11, wherein the psychiatric symptoms comprise anxiety, depression, and hallucinations.

## Patentansprüche

1. Pharmazeutische Zusammensetzung zur Verwendung bei der Vorbeugung eines Rückfalls in den Cannabiskonsum, wobei die pharmazeutische Zusammensetzung Naltrexon und Risperidon als aktive pharmazeutische Wirkstoffe umfasst.

2. Pharmazeutische Zusammensetzung zur Verwendung gemäß Anspruch 1, wobei das Massenverhältnis von Naltrexon zu Risperidon (5-30):1 beträgt.

3. Pharmazeutische Zusammensetzung zur Verwendung gemäß Anspruch 2, wobei das Massenverhältnis von Naltrexon zu Risperidon (8-15):1 beträgt.

4. Pharmazeutische Zusammensetzung zur Verwendung gemäß einem der Ansprüche 1 bis 3, ferner einen pharmazeutisch verträglichen Träger umfassend.

5. Pharmazeutische Zusammensetzung zur Verwendung gemäß einem der Ansprüche 1 bis 4, wobei die pharmazeutische Zusammensetzung zusammen mit pharmazeutisch verträglichen Hilfsstoffen verwendet wird, um eine klinisch verträgliche Formulierung herzustellen.

6. Pharmazeutische Zusammensetzung zur Verwendung gemäß Anspruch 5, wobei die Formulierung aus einer der folgenden Gruppen oder einer geeigneten Kombination davon ausgewählt ist:
1) Tabletten, Kapseln, Granulate, Pulver, dispergierbare Pulver, Suspensionen oder Lösungen; und
2) orale Flüssigkeiten, bukkale Mittel, Injektionen, Zäpfchen, Sprays, Tropfen oder Pflaster

7. Pharmazeutische Zusammensetzung zur Verwendung gemäß Anspruch 6, wobei die Formulierung in Form eines Implantats vorliegt.

8. Pharmazeutische Zusammensetzung zur Verwendung gemäß einem der Ansprüche 1 bis 7, wobei ein Subjekt ein Säugetier ist.

9. Pharmazeutische Zusammensetzung zur Verwendung gemäß Anspruch 8, wobei das Subjekt ein Mensch oder eine Ratte ist.

10. Pharmazeutische Zusammensetzung zur Verwendung gemäß einem der Ansprüche 1 bis 9 bei der Behandlung von durch Cannabissucht verursachten unerwünschten Symptomen.

11. Pharmazeutische Zusammensetzung zur Verwendung gemäß Anspruch 10, wobei die durch Cannabissucht verursachten unerwünschten Symptome psychiatrische Symptome umfassen.

12. Pharmazeutische Zusammensetzung zur Verwendung gemäß Anspruch 11, wobei die psychiatrischen Symptome Angstzustände, Depressionen und Halluzinationen umfassen.

## Revendications

1. Composition pharmaceutique pour une utilisation dans la prévention de la rechute dans la consommation de cannabis, dans laquelle la composition pharmaceutique comprend de la naltrexone et de la rispéridone en tant qu'ingrédient pharmaceutique actif.

2. Composition pharmaceutique pour une utilisation selon la revendication 1, dans laquelle le rapport massique de la naltrexone à la rispéridone est de (5-30):1.

3. Composition pharmaceutique pour une utilisation selon la revendication 2, dans laquelle le rapport massique de la naltrexone à la rispéridone est de (8-15):1.

4. Composition pharmaceutique pour une utilisation selon l'une quelconque des revendications 1 à 3, comprenant en outre un support pharmaceutiquement acceptable.

5. Composition pharmaceutique pour une utilisation selon l'une quelconque des revendications 1 à 4, dans laquelle la composition pharmaceutique est utilisée conjointement avec des substances auxiliaires pharmaceutiquement acceptables pour préparer une formulation cliniquement acceptable.

6. Composition pharmaceutique pour une utilisation selon la revendication 5, dans laquelle la formulation est choisie parmi l'un quelconque parmi les groupes suivants ou une combinaison appropriée de ceux-ci :
1) comprimés, capsules, granulés, poudres, poudres dispersables, suspensions ou solutions ; et
2) liquides oraux, agents buccaux, injections, suppositoires, sprays, gouttes ou patchs.

7. Composition pharmaceutique pour une utilisation selon la revendication 6, dans laquelle la formulation se présente sous la forme d'un implant.

8. Composition pharmaceutique pour une utilisation selon l'une quelconque des revendications 1 à 7, dans laquelle le sujet est un mammifère.

9. Composition pharmaceutique pour une utilisation selon la revendication 8, dans laquelle le sujet est un humain ou un rat.

10. Composition pharmaceutique pour une utilisation selon l'une quelconque des revendications 1 à 9 dans le traitement des symptômes indésirables causés par la dépendance au cannabis.

11. Composition pharmaceutique pour une utilisation selon la revendication 10, dans laquelle les symptômes indésirables causés par la dépendance au cannabis comprennent des symptômes psychiatriques.

12. Composition pharmaceutique pour une utilisation selon la revendication 11, dans laquelle les symptômes psychiatriques comprennent l'anxiété, la dépression et les hallucinations.
